Europäisches Patentamt

European Patent Office

Office européen des brevets

(19)

(11) Publication number: **0 251 106**
**A2**

(12) **EUROPEAN PATENT APPLICATION**

(21) Application number: 87108920.7

(22) Date of filing: 22.06.87

(51) Int. Cl.⁴: **C12N 15/00** , C12N 5/00 ,
//C12P21/02,C12P33/00,C12P1-
7/18,C12P19/44,C12P17/12

(30) Priority: 24.06.86 US 878091

(43) Date of publication of application:
07.01.88 Bulletin 88/01

(84) Designated Contracting States:
AT BE CH DE ES FR GB GR IT LI LU NL SE

(71) Applicant: MERRELL DOW
PHARMACEUTICALS INC.
2110 E. Galbraith Road
Cincinnati Ohio 45215(US)

(72) Inventor: Sunkara, Sai P.
9629 Linfield Drive
Cincinnati Ohio 45242(US)

(74) Representative: Macchetta, Francesco et al
Gruppo Lepetit S.p.A. Patent and Trademark
Department 34, Via Roberto Lepetit
I-21040 Gerenzano (Varese)(IT)

(54) Improved fusion method.

(57) The present invention relates to a method for fusing somatic cells with high efficiency which includes agglutinating the cells prior to fusion. The cells that can be fused are of animal as well as of vegetal origin. The obtained hybrid cells are capable of producing useful products.

EP 0 251 106 A2

# IMPROVED FUSION METHOD

The fusion of mouse myeloma cells to spleen cells from immunized mice by Kohler and Milstein in 1975 (Nature 256 (1975), 495-497) demonstrated for the first time that it was possible to obtain a continuous cell line making homogeneous (so-called "monoclonal") antibody. Since this seminal work, much effort has been directed to the production of various hybrid cells (called "hybridomas") and to the use of antibodies made by these hybridomas for various scientific investigations and diagnostic purposes in medicine. The method for preparing the hybridoma generally comprises the following steps:

a) Immunizing an appropriate animal, e.g. mice or rats, with an immunogenic agent. The immunization protocol should be such as to produce useful quantities of suitably primed splenocytes.

b) Removing spleens from the immunized animal and making a spleen suspension in an appropriate medium.

c) Fusing the suspended spleen cells with myeloma cells from a suitable cell line in the presence of a suitable fusion promoter. The fusion promoter could be Sendai virus, a chemical fusogen, e.g., polyethylene glycol (PEG) having an average molecular weight of about 1000 to about 4000 (commercially available as PEG 1000, etc.). Another technique which is useful in effecting fusion is the electro-fusion according to the known methods such as those utilized by Zimmermann and Scheurich, Planta, 151 (1981), 26-32, Vienken et al., Planta, 157 (1983), 331, and Jacob et al., Sbud. Biophys., 94 (1983), 99. The myeloma cell line used should preferably be of the so-called "drug-resistant" type, so that unfused myeloma cells will not survive in a selective medium, while hybrids will. The most common class is 8-azaguanine resistant cell lines which lack the enzyme hypoxanthine guanine phosphoribosyl transferase and hence will not be supported by HAT (hypoxanthine, aminopterin, and thymidine) medium.

d) Diluting and culturing in separate containers the mixture of unfused spleen cells, unfused myeloma cells and fused cells in the selective medium which will not support the unfused myeloma cells for a time sufficient to allow death of unfused cells (about one week). The dilution may be a type of limiting one, in which the volume of diluent is statistically calculated to isolate a certain number of cells (e.g. each well of a microtiter plate). In the selective medium the unfused myeloma cells perish. Since the unfused spleen cells are non-malignant they have only a finite number of generations. Thus, after a certain period of time (about one week) these unfused spleen cells fail to reproduce. The fused cells, on the other hand, continue to reproduce because they possess the malignant quality of the myeloma parent and the ability to survive in the selective medium of the spleen cell parent.

e) Evaluating the supernatant in each container (well) containing a hybridoma for the presence of antibody directed against the antigene originally used.

f) Selecting (e.g. by limiting dilution) and cloning hybridomas producing the desired antibody.

Once the desired hybridoma has been selected and cloned, the resultant antibody may be produced in one of two ways. The purest monoclonal antibody is produced by in vitro culturing of the desired hybridoma in a suitable medium for a suitable length of time, followed by recovery of the desired antibody from the supernatant. The suitable medium and suitable length of culturing time are known or are readily determined. This in vitro technique produces essentially monospecific monoclonal antibody, essentially free from other specific antihuman immune globulin. There is a small amount of other immune globulin present since the medium contains xenogeneic serum (e.g. fetal calf serum). However, this in vitro method may not produce a sufficient quantity or concentration of antibody for some purposes, since the concentration of monoclonal antibody is only about 50 $\mu$g/ml.

To produce a much greater concentration of slightly less pure monoclonal antibody, the desired hybridoma may be injected into mice, preferably syngenic or semi-syngenic mice. The hybridoma will cause formation of antibody-producing tumors after a suitable incubation time, which will result in a high concentration of the desired antibody (about 5-20 mg/ml) in the bloodstream and peritoneal exudate (ascites) of the host mouse.

This method is extensively disclosed in the literature. It has particular and serious drawbacks. PEG has gained increasing acceptance as a fusogen because it is available in pure form and in the desired molecular weight range, and because of its easy handling compared to the Sendai virus. However, the range of concentrations over which PEG is effective is very narrow. The optimal concentration is 50 ± 5% weight/volume. At this concentration it exhibits significant cytotoxic effects against all cells to be fused and against the fused hybrid cells. The result is a reduction in the number of viable hybrid cells. When using suboptimal concentration of PEG, on the other hand, only a moderate cytotoxicity is observed, but the fusion efficiency and consequently the formation of hybridoma is low. In contrast to the Sendai virus, the known chemical fusogens such as polyethylene glycol (PEG) and electro-fusion do not effect agglutination of the cells to be fused so that the cell membranes are not in close proximity efficient for fusion.

Thus, one object of the invention is to develop an improved method for fusing cells, i.e., mammalian cells or plant cells capable of producing a useful product, e.g. immunized spleen cells, with a suitable fusion partner capable of continuous propagation, e.g. myeloma cells or other permanent cells, according to the above discussed method which permits agglutination of the cells to be fused. Another object of this invention is to reduce the cytotoxic effect of conventional chemical fusogens such as PEG, lysolecithin, dextran, DMSO, polyvinyl alcohol, poly-L-ornithin and salts known to be useful such as sodium nitrate or a combination thereof. In the fusion process this results in a higher yield of the desired hybrid cells. Another object is to produce agglutinated cells to fused by electro-fusion techniques.

These and other objects will become apparent from the following description. The present invention in its generic concept relates to a method for preparing a hybrid cell by first agglutinating the cells to be fused by action with an agglutinogen. More particularly, the present invention relates to an improvement of the method for preparing a hybrid cell by fusing a mammalian cell capable of producing a useful product with a mammalian cell capable of continuous propagation under selective conditions which comprises contacting a mixture of cells to be fused with an effective amount of an agglutinogen and thereafter subjecting the agglutinated cells to fusion. In another aspect the invention relates to the agglutination and fusion of plant cells to produce hybrid cells capable of producing useful products. Thus the present invention also relates to a method for preparing a fused hybrid çell which comprises contacting a plant cell capable of producing a useful product and a plant cell capable of continuous propagation with an effective amount of an agglutinogen and thereafter subjecting the agglutinated cells to fusion.

An "agglutinogen" is a term used to describe any agent capable of agglutinating the cells to be fused. Although all agglutinogens are embraced typical agglutinogens are phytohaemagglutinin (PHA), concanavallin A and peanut agglutinin. The preferred agglutinogen according to the present invention is PHA. The concentration of the agglutinogen should be adjusted such that no cytotoxic effects are observed and agglutination occurs to a significant degree.

A suitable range of the agglutinogen PHA is 25 - 400 μg/ml. The preferred concentration of PHA is about 150 - 200 μg/ml with 200 mg/ml being most preferred. The suitable concentration of the other agglutinogens may be readily determined by standard techniques well known in the art. Agglutination treatment is carried out at physiological temperatures, e.g., 37°C and for a sufficient length of time, e.g., 5 - 15 min. after sufficient agglutination has occurred, the cells are subjected to fusion. In one embodiment, a conventional chemical fusogen, preferably PEG is added and the mixture is incubated under physiological conditions to effect fusion of the agglutinated cells. When using PEG, the concentration is about 30 - 50% weight/volume. The preferred concentration is about 40 ± 5% weight/volume. The optimal fusion time is about 1 min. Longer times can be used with the risk of cytotoxicity increasing with increased time, particularly above 2 min. In another embodiment fusion of the agglutinated cells is effected by electro-fusion.

This novel fusion technique of the present invention results in significantly increased viable hybrid cell formation compared to prior art results.

The term "useful product" embraces all products a mammalian cell or a plant cell to be fused may produce. Typical examples are biologically active proteins, glycoproteins, polypeptides, enzymes and non-protinaceous compounds such as alkaloids, steroids, diosgenin, anthraquinones, pyrethrins, essential oils, polysaccharides, cardiac glycosides, perfume and cosmetic components.

Typical examples of biologically active substances that can be produced from the cells to be fused are shown in the following table I to V.

## TABLE I

| | |
|---|---|
| Alkaloids | Insecticides |
| Allergens | Latex |
| Anthraquinones | Lipids |
| Antileukaemic agents | Naphthoquinones |
| Antitumour agents | Nucleic acids |
| Antiviral agents | Nucleotides |
| Aromas | Oils |
| Benzoquinones | Opiates |
| Carbohydrates (including polysaccharides) | Organic acids |
| Cardiac glycosides | Peptides |
| Chalcones | Perfumes |
| Dianthrenes | Phenols |
| Enzymes | Pigments |
| Enzyme inhibitors | Plant growth regulators |
| Flavanoids, flavones | Proteins |
| Flavours (including sweeteners) | Steroids and derivatives |
| Fluranocoumarins | Sugars |
| Hormones | Tannis |
| | Terpenes and terpenoids |
| | Vitamins |

TABLE II

| Biologically Active Substance | Cell Source | Use |
| --- | --- | --- |
| Insulin | Pancreatic islet cells | Antidiabetic |
| Glucagon | Pancreatic islet cells | Regulates the insulin production |
| Somatostatin | Pancreatic islet cells | Regulates the insulin production |
| ACTH | Anterior pituitary cells | Anti-inflammatory |
| Lutenizing hormone | Pituitary cells | Regulate reproduction |
| Follicle stimulating hormone | Pituitary cells | Regulate reproduciton |
| Growth hormone | Pituitary cells | Promotes growth |
| Lutenizing Hormone- Release Hormone | Pituitary cells | Regulates fertility |
| Prolactin | Pituitary cells | Stimulates milk production |
| Thyrotropin (TSH) | Pituitary cells | Hypothyrodism |
| Thymic hormones | Thymus cells | Immunomodulator |
| Erythropoietin | Kidney | Stimulates erythropoiesis |
| Epidermal growth factor | Submaxillary gland | Promotes cells proliferation and inhibits gastric acid secretion |
| Oxytocin | Posterior pituitary | Controls uterine contraction and milk production |
| Vasopressin | Posterior pituitary | Antidiuretic hormone |
| Enkephalin | Adrenal chromaffin cells | Analgesic |

0 251 106

TABLE II (Continued)

| | | |
|---|---|---|
| (ANF) Atrial Naturietic Factor | Heart cells | Vasodialator |
| $\beta$-endorphins | Brain, pituitary gland | Analgesic |
| Inhibin | Granulosa cells (ovary) | Fertility control |
| Calcitonin | Thyroid cells | Increases deposition of $Ca^{++}$ in bones |
| Parathyroid hormone | Parathyroid (principal cell) | Causes resorption of bone |
| Interleukin-1 | Macrophage | Antitumor |
| Interleukin-2 | T helper cell | Antitumor |
| Interleukin-3 | T helper cell | Stem cell proliferation |
| $\alpha$-Interferon | Leukocytes | Antiviral, antitumor |
| Colony stimulating factor-2 | T cell | MQ and granulocyte proliferation |
| CSF-1 | Fibroblasts | MQ growth and activation |
| B cell growth factor | T helper cell | Immunodefficiency |
| Tumor necrosis factor | Macrophage | Antitumor |
| $\gamma$-Interferon | Macrophage, T helper cells | Antiviral and antitumor |
| $\beta$-Interferon | Fibroblast | Antiviral and antitumor |
| Macrophage activating factor | Macrophage | Antitumor and antiviral |
| Angiogenin | Carcinoma cells | Wound healer, vascularlization |
| Steroids | Adrenal cortica | Reproduction |
| Nerve growth factor | Salivary gland cells | Neuronal regeneration |
| Platlet derived growth factor | (Endothine platlet) | Wound healing |
| Melanophore stimulating hormone and melatonin | Pinearocyte | Pigmentation control |

TABLE III

| Compound group | Type and examples |
|---|---|
| Pharmaceuticals | Alkaloids, steroids, anthraquinones |
| Enzymes | Proteases (e.g., papain) |
| Latex | Isoprenoids (e.g., rubber) |
| Waxes | Wax esters (e.g., jojoba) |
| Pigments | Stains and dyes |
| Oils | Fatty acids (e.g., seed oils) |
| Agrochemicals | Insecticides (e.g., pyrethrins) |
| Cosmetic substances | Essential oils (e.g., monoterpenes) |
| Food additives | Flavour compounds, non-nutritive sweeteners (e.g., thaumatin) |
| Gums | Polysaccharides (e.g., gum arabic) |

TABLE IV

| Industry | Plant product | Plant species | Industrial uses |
|---|---|---|---|
| Pharmaceuticals | Codeine (alkaloid) | Papaver somniferum | Analgesic |
| | Diogenin (steroid) | Dioscorea deltoidia | Anti-fertility agents |
| | Quinine (alkaloid) | Cinchona ledgeriana | Antimalarial |
| | Digoxin (caridac glycoside) | Digitalis lanata | Cardiatonic |
| | Scopolamine (alkaloid) | Datura stramonium | Antihypertensive |
| | Vincristine (alkaloid) | Catharanthus roseus | Antileukaemic |
| Agrochemicals | Pyrethrin | Chrysanthemum cinerariaefolium | Insecticide |
| Food and drink | Quinine (alkaloid) | Cinchona ledgeriana | Bittering agent |
| | Thaumatin (chalcone) | Thaumatococcus danielli | Non-nutritive sweetener |
| Cosmetics | Jasmine | Jasminum sp. | Perfume |

- TABLE V

| Medicinal agent | Activity | Plant source |
| --- | --- | --- |
| Steroids from diosgenin | Anti-fertility agents | Dioscorea deltiodea |
| Codeine | Analgesic | Papaver somniferum |
| Atropine | Anticholinergic | Atropa belladonna |
| Reserpine | Antihypertensive | Rauwolfia serpentina |
| Hyoscyamine | Anticholinergic | Hyoscyamus niger |
| Digoxin | Cardiatonic | Digitalis lanata |
| Scopolamine | Anticholinergic | Datura metel |
| Digitoxin | Cardiovascular | Digitalis purpurea |
| Pilocarpine | Cholinergic | Pilocarpus jabonandi |
| Quinidine | Antimalarial | Cinchona ledgeriana |

Eukaryotic cells of mammalian and plant origin may be used, plant cells preferably in the form of protoplasts. Protoplast formation is well known by those of ordinary skill in the art.

Furthermore, cells may be used which produce biologically active substances other than proteins such as steroid hormones. In the case of plant cells, cells may be used which produce alkaloids such asquinine, reserpine, cocaine, atropine, scopolamine, digitalis, morphine-like substances.

Furthermore, cells may be used which produce essential oils and scents such as muscone, civetone, geraniol and other terpene-like substances useful in the perfume industry.

Examples of continuously propagating (permanent) cells to be used as fusion partners and for the immortalization of mammalian and plant cells are myeloma cells of various origin such as mouse, rat or human origin. Typical examples of permanently growing cells are tumor cells such as myeloma cells, cells derived from human neoplasia including HeLa cells, HE p-2 derived from pharynx cancer cells and KB derived from rhino-pharyngo cancer cells, lymphoblastoid cells, Epstein-Barr virus transformed cells, highly propagating embryo cells, hepatoma cells, renal carcinoma cells. Of particular importance for the present invention are drug resistant myeloma cells, including murine and human myeloma cells. It is also generally preferred that the myeloma cells used be of the so-called "non-secreting" type, although secreting types may be used.

Typically, continuously growing plant cells are tumorous crown gall cells, i.e., plant cells transformed by a virulent strain of Agrobacterium tumefaciens and protoplasts thereof.

The fused cells are grown or propagated in conventional nutrient media containing conventional sources of carbon, nitrogen and mineral salts. The propagation is carried out generally under aerobic conditions, i.e., in the presence of a mixture of 95% oxygen and 5% carbon dioxide. It is evident that propagation and all preceding steps are carried out under sterile conditions. Larger amounts of hybrid cells may be produced in syngenic or athymic nude mice according to conventional methods. The cells can be harvested in the form of ascites or solid tumors and propagated as conventional cell cultures. Such cell cultures can be induced to produce larger amounts of the desired products. A typical example is the induction of hybrid cells derived from Langerhans islet cells induced with glucose to form increased amounts of insulin.

In an analogous manner, the production of urokinase can be stimulated by adding glycine to urokinase producing hybrid cells.

The isolation of the useful product produced by the hybrid cells is carried out in any conventional manner, e.g., by removing the nutrient medium from the hybrid cells and by extraction, counter-current distribution, affinity chromatography, precipitation, gel filtration, ion exchange chromatography, HPLC, etc., and a combination of these methods.

The products are in general well known and identified and their use therefore is also well known.

The invention is explained in detail in the following examples:

## EXAMPLE 1

$1 \times 10^8$ spleen cells from 2 spleens of Balb/c mice are mixed with $1 \times 10^7$ mouse myeloma cells (Fox - NY). Wash the cell population once in 15 ml tube with sterile Hank solution and centrifuge. Gently resuspend (vortex) the so-obtained pellet in the remaining solution, add 0.2 ml of PHA solution (200 $\mu$g/ml) and incubate for 10 min. at 37°C. At the end of incubation add to the tube 0.8 ml of PEG 50% weight/volume (average molecular weight 1000) solution to obtain a final concentration of 40% PEG. After 1 min. incubation add 10 ml of complete medium (MEM with 10% fetal calf serum) and further incubate for 1 hr. at 37°C in a $CO_2$ incubator. At the end of incubation centrifuge the cells and aspirate the medium containing PHA and PEG. Suspend the pellet in 20 ml of HAT medium. Distribute the cell suspension so-obtained into 96 well plates (10 plates) at 0.02 ml/well and incubate the plates at 37°C in a $CO_2$ incubator. Change the HAT medium once a week. In about 2 weeks viable hybrid cells can be observed under a microscope in some of the wells.

## EXAMPLE 2

In accordance with the procedure of Example 1 spleen cells of Balb/c mice are used which have previously been immunized with hepatitis B virus according to the method disclosed by Wands and Zurawski Jr. in U.S. Patent 4,271,145. Hybrid cells are isolated which produce monoclonal antibodies to antigenic determinants of hepatitis B virus.

EXAMPLE 3

Activated peritoneal macrophages obtained from Corynebacterium parvum treated mice are fused with mouse myeloma cells according to the procedure of Example I. The hybrid cells obtained are screened for interleukin I, γ-interferon, tumor necrosis factor and macrophage activating factor.

EXAMPLE 4

T-lymphocytes obtained from mouse spleens are activated by mixed lymphocyte reaction and then fused as described in Example I. The obtained cell hybrids are screened for interleukin 2, 3 and B cell growth factor.

EXAMPLE 5

Cells from the pituitary gland are fused as described in Example I and the obtained cell hybrids are screened for growth hormones and prolactin utilizing hormones.

EXAMPLE 6

Protoplasts from crown gall tumors (induced by a virulent strain of Agrobacterium tumefaciens of a tobacco plant) are fused with protoplasts obtained from Rauwolfia serpentina in accordance with the procedure of Example I and the rapidly dividing hybrid cells are extracted by established procedures to obtain reserpine.

EXAMPLE 7

Protoplasts from crown gall tumors of a tobacco plant are fused with protoplasts obtained from Digitalis purpurea in accordance with the procedure of Example I and the so-obtained hybrid cells are extracted to obtain a mixture of digitalis glycosides.

EXAMPLE 8

Protoplasts of crown gall tumors of a tobacco plant are fused with protoplasts obtained from Atropa belladonna in accordance with the procedure of Example I and the so-obtained hybrid cells are extracted to obtain atropine.

Claims

I. A method for preparing a hybrid cell by fusing a mammalian cell capable of producing a useful product with a mammalian cell capable of continuous propagation under selective conditions which comprises contacting a mixture of cells to be fused with an effective amount of an agglutinogen and thereafter subjecting the agglutinated cells to fusion.

2. A method for preparing a fused hybrid cell which comprises contacting a plant cell capable of producing a useful product and a plant cell capable of continuous propagation with an effective amount of an agglutinogen and thereafter subjecting the agglutinated cells to fusion.

3. A method according to Claim I or 2, wherein the agglutinogen is phytohaemagglutinin.

4. A method according to Claim I or 2, wherein the fusion is effected by chemical fusion.

5. A method according to Claim I or 2, wherein the fusion is effected by electro-fusion.

6. A method according to Claim 4, wherein the chemical fusogen if PEG having an average molecular weight from about I000 to about 4000.

7. The fused hybrid cell produced according to Claim I or 2.